(19) Europäisches Patentamt
European Patent Office
Office européen des brevets

(11) **EP 2 751 254 B1**

(12) **EUROPEAN PATENT SPECIFICATION**

(45) Date of publication and mention
of the grant of the patent:
**13.12.2017 Bulletin 2017/50**

(51) Int Cl.:
*C12N 1/00* (2006.01)      *C12N 1/04* (2006.01)
*C12P 21/02* (2006.01)      *C12N 1/20* (2006.01)
*A23P 20/12* (2016.01)      *A23L 29/212* (2016.01)
*A23L 33/135* (2016.01)     *A23C 9/12* (2006.01)

(21) Application number: **12772120.7**

(22) Date of filing: **31.08.2012**

(86) International application number:
**PCT/GB2012/052149**

(87) International publication number:
**WO 2013/030596 (07.03.2013 Gazette 2013/10)**

(54) **IMPROVED VIABILITY OF PROBIOTIC MICROORGANISMS USING POLY - GAMMA - GLUTAMIC ACID**

VERBESSERTE LEBENSFÄHIGKEIT VON PROBIOTISCHEN MIKROORGANISMEN MIT POLY-GAMMA-GLUTAMINSÄURE

VIABILITÉ AMÉLIORÉE DE MICRO-ORGANISMES PROBIOTIQUES EN UTILISANT ACIDE GLUTAMIQUE POLY GAMMA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2011 GB 201115143**

(43) Date of publication of application:
**09.07.2014 Bulletin 2014/28**

(73) Proprietor: **The University of Wolverhampton Wolverhampton, West Midlands WV1 1LY (GB)**

(72) Inventors:
• **RADECKA, Iza**
  **Wolverhampton**
  **West Midlands WV1 1LY (GB)**
• **HILL, David**
  **Wolverhampton**
  **West Midlands WV1 1LY (GB)**
• **BARTLETT, Terence**
  **Wolverhampton**
  **West Midlands WV1 1LY (GB)**
• **BHAT, Aditya**
  **Aberystwyth**
  **SY23 2DR (GB)**
• **KEDIA, Gopal**
  **Birmingham**
  **West Midlands B15 2EE (GB)**

(74) Representative: **Trueman, Lucy Petra**
**Barker Brettell LLP**
**100 Hagley Road**
**Edgbaston**
**Birmingham B16 8QQ (GB)**

(56) References cited:
**KR-B1- 101 000 364**

• **SHANTANU S. BALKUNDI ET AL: "Encapsulation of Bacterial Spores in Nanoorganized Polyelectrolyte Shells +", LANGMUIR, vol. 25, no. 24, 15 December 2009 (2009-12-15), pages 14011-14016, XP055046449, ISSN: 0743-7463, DOI: 10.1021/la900971h**
• **ISHWAR BAJAJ ET AL: "Poly (glutamic acid) An emerging biopolymer of commercial interest", BIORESOURCE TECHNOLOGY, ELSEVIER BV, GB, vol. 102, no. 10, 10 February 2011 (2011-02-10), pages 5551-5561, XP028407803, ISSN: 0960-8524, DOI: 10.1016/J.BIORTECH.2011.02.047 [retrieved on 2011-02-15]**
• **DATABASE WPI Week 200774 Thomson Scientific, London, GB; AN 2007-787673 XP002688573, & CN 1 948 463 A (UNIV HUAZHONG AGRIC) 18 April 2007 (2007-04-18)**

- BURGAIN J ET AL: "Encapsulation of probiotic living cells: From laboratory scale to industrial applications", JOURNAL OF FOOD ENGINEERING, BARKING, ESSEX, GB, vol. 104, no. 4, 29 December 2010 (2010-12-29), pages 467-483, XP028173566, ISSN: 0260-8774, DOI: 10.1016/J.JFOODENG.2010.12.031 [retrieved on 2011-01-05]

- MOHAMMAD ARIFUL ISLAM ET AL: "Microencapsulation of Live Probiotic Bacteria", JOURNAL OF MICROBIOLOGY AND BIOTECHNOLOGY, vol. 20, no. 10, 28 October 2010 (2010-10-28), pages 1367-1377, XP055046708, ISSN: 1017-7825, DOI: 10.4014/jmb.1003.03020

**Description**

The present invention relates to the use of poly-γ-glutamic acid (γ-PGA) in improving the viability of probiotic microorganisms. Methods of manufacturing γ-PGA, in particular γ-PGA with specific properties, are disclosed but not claimed.

Probiotics were defined by the World Health Organisation in 2002 as live microorganisms that when administered in adequate amounts confer a health benefit on the host. Accordingly probiotic microorganisms have been introduced into a variety of food and drink products for administration to a human or animal. However, many of the probiotic microorganisms used in food and drink products do not survive for long enough to confer a health benefit on the host. The processes that they must be subjected to to get them into the food or drink product and the human or animal body result in many becoming unviable before they have the chance to affect the host in any way.

Firstly the probiotic microorganisms are subjected to a freeze drying process. Secondly the probiotic microorganisms are incorporated into a food or drink product and then stored for a period of time that depends on the product they have been incorporated into. Finally the probiotic microorganisms are ingested by a human or animal with the food or drink product; the conditions of the mouth and stomach in particular being adverse to the viability of the probiotic microorganisms. Accordingly having passed through all of the above processes there are only relatively few microorganisms remaining viable and able to exert some effect on the host.

Furthermore the nature of the food or drink product into which the probiotic microorganism is introduced is generally limited to milk based products which maintain a weak acid environment tolerated by the microorganisms. However, even under these conditions, shelf life is restricted.

Therefore, there remains a need for a means to improve the viability of probiotic microorganisms as they pass through, in particular, the freeze drying, storage and ingestion processes. It would also be useful to be able to put the microorganisms into food and drink products that are not milk based.

KR 101 000 364 discloses a lactobacillus particle which is double-coated with chitosan and poly-gamma-glutamic acid. SHANTANU S. BALKUNDI ET AL (LANGMUIR, (20091215), vol. 25, no. 24) describes encapsulation of bacterial spores in nanoorganized polyelectrolyte shells. The shells were constructed on *Bacillus subtilis* spores using biocompatible polymers polyglutamic acid, polylysine, albumin, lysozyme, gelatin A, protamine sulfate, and chondroitin sulfate.

Accordingly the present invention provides an ingestible product comprising a probiotic microorganism at least partially coated directly with a poly glutamic acid, wherein the poly glutamic acid is poly-γ-glutamic acid (γ-PGA).

The γ-PGA preferably has a molecular weight of from 10,000 to 1,000,000 Daltons (Da). The γ-PGA may have a molecular weight of from 20,000 to 800,000 Da or 20,000 to 700,000 Da or 20,000 to 600,000 Da. More preferably the γ-PGA may have a molecular weight of from 50,000 to 500,000 Da, for example 100,000 to 300,000 Da or 200,000 to 260,000 Da.

The γ-PGA may be produced in any suitable bacteria, for example *Bacillus subtilis* and *Bacillus licheniformis.* In one embodiment the γ-PGA is produced in *Bacillus subtilis,* for example *Bacillus subtilis* natto.

The probiotic microorganism may be a bacteria. Examples of possible bacteria include *Bifidobacterium longum, Bifidobacterium breve* and *Lactobacillus casei.*

All or part, of some or all, of the probiotic microorganisms may be coated with biopolymer, for example by suspending the probiotic microorganisms in biopolymer with subsequent freeze drying.

All or part, of some or all, of the probiotic microorganisms may be coated with the poly glutamic acid, for example by suspending the probiotic microorganisms in the polyglutamic acid with subsequent freeze drying

The ingestible product may be a food or beverage product. The ingestible product may be milk based, for example yogurt or a yogurt drink, or non-milk based, for example fruit based such as a fruit juice.

Also provided is a probiotic microorganism at least partially coated directly with a poly glutamic acid wherein the poly glutamic acid is poly-γ-glutamic acid (γ-PGA).

The γ-PGA preferably has a molecular weight of from 10,000 to 1,000,000 Daltons (Da). The γ-PGA may have a molecular weight of from 20,000 to 800,000 Da or 20,000 to 700,000 Da or 20,000 to 600,000 Da. More preferably the γ-PGA may have a molecular weight of from 50,000 to 500,000 Da, for example 100,000 to 300,000 Da or 200,000 to 260,000 Da.

The γ-PGA may be produced in any suitable bacteria, for example *Bacillus subtilis* and *Bacillus licheniformis.* In one embodiment the γ-PGA is produced in *Bacillus subtilis,* for example *Bacillus subtilis* natto.

The probiotic microorganism may be a bacteria. Examples of possible bacteria include *Bifidobacterium longum, Bifidobacterium breve* and *Lactobacillus casei.*

Also provided is method of at least partially coating directly a probiotic microorganism with a poly glutamic acid comprising mixing the microorganism with a solution of a poly glutamic acid, wherein the poly glutamic acid is poly-γ-glutamic acid (γ-PGA).

The poly glutamic acid may be provided in a concentration of from 2 to 15% (w/v), for example from 5 to 12% (w/v), such as 10% (w/v).

The microorganism may be provided in cell pellet form.

The probiotic microorganism may be a bacteria. Examples of possible bacteria include *Bifidobacterium longum, Bifidobacterium breve* and *Lactobacillus casei.*

The γ-PGA preferably has a molecular weight of from 10,000 to 1,000,000 Daltons (Da). The γ-PGA may have a molecular weight of from 20,000 to 800,000 Da or 20,000 to 700,000 Da or 20,000 to 600,000 Da. More preferably the γ-PGA may have a molecular weight of from 50,000 to 500,000 Da, for example 100,000 to 300,000 Da or 200,000 to 260,000 Da.

The γ-PGA may be produced in any suitable bacteria, for example *Bacillus subtilis* and *Bacillus licheniformis.* In one embodiment the γ-PGA is produced in *Bacillus subtilis,* for example *Bacillus subtilis* natto.

A method of making poly-γ-glutamic acid (γ-PGA) is disclosed but not claimed, the method comprising the steps of:

a) preparing a starter culture of the appropriate bacterial colony in fermentation broth;
b) adding starter culture to a growth medium;
c) producing γ-PGA in the growth medium.

In the context of this method the "starter culture" comprises one or more colonies of an appropriate strain of bacteria, preferably one or more highly mucoid colonies, inoculated in a growth medium, for example, TSB and incubated, for example at 37°C for 24h.

The bacterial colony may be *B. subtilis* or *B. licheniformis.* The bacterial colony is preferably *B. subtilis* natto.

The fermentation broth may be any suitable broth, for example tryptone soy broth (TSB).

The growth media may be any suitable growth media, for example growth media E or growth media GS.

Growth medium GS generally produces a higher yield of γ-PGA than growth medium E. The growth media can affect the crystallinity of the γ-PGA with the γ-PGA produced in medium E being amorphous and the γ-PGA produced in medium GS being crystalline. Furthermore the growth medium has an effect on the formation of the salt or free acid form of the γ-PGA. In growth medium GS most of the γ-PGA produced was the sodium salt whereas in growth medium E a considerable amount of the acid form was produced. Molecular weight is affected by both growth medium and strain of bacteria.

The bacterial colony may be highly mucoid.

The fermentation step may take place at a temperature of 35 to 39°C, for example 37°C.

The fermentation time may be from 18 to 30 hours, for example from 20 to 28 hours, such as 24 hours.

The growth step may take place at a temperature of 35 to 39°C, for example 37°C.

The growth time may be from 90 to 100 hours, for example from 94 to 98 hours, such as 94 hours.

The growth step may include the steps of agitating in any suitable fermenter vessel for all or part of the growth time.

Agitation can range from 100 rpm to 1000 rpm.

Any suitable fermenter vessel could be used including, for example, shake flasks, aerated stirred tank reactors and solid state fermenters.

The poly-γ-glutamic acid may be isolated from the growth media. The poly-γ-glutamic acid in the growth media may first be subjected to centrifugation.

Any suitable alcohol based solvent, such as ethanol, may then be added to the cell free supernatant resulting from centrifugation, for example at a ratio of 2:1 to 6:1, for example 4:1, alcohol to supernatant. The alcohol/supernatant mixture may be incubated at 2 to 6°C, for example 4°C for 70 to 75 hours, for example 72 hours.

The poly-γ-glutamic acid may be removed from the alcohol/supernatant mixture by centrifugation and/or filtration.

The poly-γ-glutamic acid may be subjected to lyophilization. The poly-γ-glutamic acid may be frozen before being subjected to lyophilization

Aspects of the invention will now be described by reference to experimental work carried out. The experimental work is purely illustrative of the aspects of the invention and is in no way intended to be limiting on the scope of the invention.

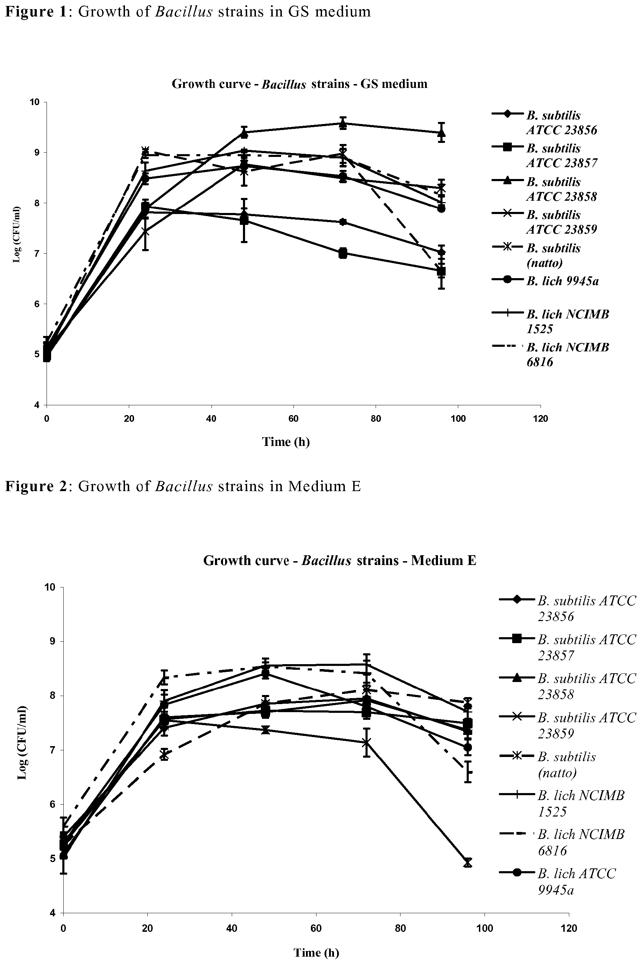

**Figure 1** shows the growth of the *Bacillus* strains in GS medium;

**Figure 2** shows the growth of the *Bacillus* strains in E medium;

**Figure 3** shows FT-IR spectra for the γ-PGA produced by the *Bacillus* strains compared to that of a commercially available γ-PGA sample;

**Figure 4** shows crude yield of γ-PGA from different *Bacillus* strains in growth media E and GS;

**Figure 5** shows the XRD spectra for the strain - *B. subtilis* ATCC 23856 in GS and E medium;

**Figure 6** shows ICP-AES results showing % salt composition of γ-PGA produced by the different *Bacillus* strains in GS medium;

**Figure 7** shows ICP-AES results showing % salt composition of γ-PGA produced by the different *Bacillus* strains in E medium;

**Figure 8** shows the effect of pressure cooked γ-PGA and sucrose on viability of probiotic bacteria during freeze drying;

**Figures 9a and b** show the effect of γ-PGA on the viability of *B. longum* and *B. breve* in orange juice; and

**Figures 10a and b** show the effect of γ-PGA on the viability of *B. longum* and *B. breve* in simulated gastric juice.

**PART 1. PRODUCTION OF POLY-γ-GLUTAMIC ACID FROM EIGHT DIFFERENT STRAINS OF *BACILLUS* (REFERENCE).**

This experiment describes the production of γ-PGA with 8 different strains of bacteria - *B.subtilis* natto, *B. subtilis* ATCC 23856, *B. subtilis* ATCC 23857, *B. subtilis* ATCC 23858, *B. subtilis* ATCC 23859, *B. licheniformis* 9945a, *B. licheniformis* NCIMB 1525 and *B. licheniformis* NCIMB 6816 in shake flasks.

### 1. Introduction

It is known from previous research that *Bacillus subtilis* and *Bacillus licheniformis* are able to produce extracellular γ-PGA which can be easily recovered from the production medium. In this work, five strains *of Bacillus subtilis* - *B. subtilis* natto, *B. subtilis* subsp. subtilis ATCC 23856 (also known as *B. subtilis* subsp. subtilis SB19 EMG50), *B. subtilis* ATCC 23857 (also known as *B. subtilis* subsp. subtilis 168 EMG51), *B. subtilis* ATCC 23858 (also known as *B. subtilis* subsp. subtilis EMG52), *B. subtilis* ATCC 23859 (also known as *B. subtilis* subsp. subtilis EMG53) and three strains of *Bacillus licheniformis* - *B. licheniformis* 9945a, *B. licheniformis* NCIMB 1525 (also known as *B. licheniformis* 1229) and *B. licheniformis* NCIMB 6816 (also known as *B. licheniformis* Glaxo417) were investigated for the production of γ-PGA.

To try to obtain γ-PGA with different properties, such as crystallinity, form of peptide and yield along with molecular weight, two different media were used for production.

### 2. Material and methods

2.1 Bacterial strains

All strains - *B. subtilis* natto, *B. subtilis* ATCC 23856, *B. subtilis* ATCC 23857, B. *subtilis* ATCC 23858, *B. subtilis* ATCC 23859, *B. licheniformis* 9945a, *B. licheniformis* NCIMB 1525 and *B. licheniformis* NCIMB 6816 - were obtained from National Collection of Industrial and Marine Bacteria (NCIMB). The stock cultures were freeze-dried and stored at -20°C. Before use, cultures were revived aseptically and grown on general purpose microbiology media overnight at 37°C.

### 2.2 Growth media

Tryptone soya agar (TSA), tryptone soya broth (TSB) and one-quarter strength ringer solution were prepared according to the manufacturer's protocol (Lab M, UK).

The composition of GS medium and Medium E has been given in Table 1 & 2 below. The pH of both media was adjusted to 7.2 using 3 M NaOH and 1 M HCl.

All media were prepared using de-ionised water and sterilized at 121°C at 15 p.s.i. for 20 min. Sucrose solution was sterilised separately in a pressure cooker (110°C at 5 p.s.i. for 30 min) and vitamin solution was filter sterilised (0.2 μm Ministart) and added separately to GS medium.

**Table 1:** Composition of GS medium

| Chemical | Amount | Source |
| --- | --- | --- |
| L-glutamic acid | 20g/L | Sigma-Aldrich |
| Sucrose | 50g/L | Acros Organics |

(continued)

| Chemical | Amount | Source |
|---|---|---|
| Potassium dihydrogen orthophosphate, $KH_2PO_4$ | 2.7g/L | Fisher Scientific |
| Di-sodium hydrogen phosphate anhydrous, $Na_2HPO_4$ | 4.2g/L | AnalaR |
| Sodium chloride, NaCl | 50g/L | Aldrich Chemical Co. Ltd |
| Magnesium sulphate heptahdrate, $MgSO_4 \cdot 7H_2O$ | 5g/L | AnalaR |
| Murashige-Skoog vitamin solution | 1ml/L | Sigma-Aldrich |

**Table 2:** Composition of Medium E

| Chemical | Amount | Source |
|---|---|---|
| L-glutamic acid | 20g/L | Sigma-Aldrich® |
| Citric acid | 12g/L | Acros Organics |
| Glycerol | 80g/L | Avocado |
| Ammonium chloride, $NH_4Cl$ | 7g/L | Riedel-de Haën |
| Magnesium sulphate heptahdrate, $MgSO_4 \cdot 7H_2O$ | 0.5g/L | AnalaR |
| Iron (III) chloride hexahydrate, $FeCl_3 \cdot 6H_2O$ | 0.2g/L | Sigma-Aldrich® |
| Di-potassium hydrogen orthophosphate anhydrous, $K_2HPO_4$ | 0.5g/L | AnalaR (BDH) |
| Calcium chloride dehydrate, $CaCl_2 \cdot 2H_2O$ | 0.15g/L | Fisher Chemicals |
| Manganese (II) sulphate monohydrate, $MnSO_4 \cdot H_2O$ | 0.2g/L | Sigma |

**2.3 Production of γ-PGA**

Production of γ-PGA with each bacterial strain was done in triplicates. Highly mucoid colonies of the appropriate strain were selected and inoculated in flasks containing 250 ml TSB and incubated at 37°C for 24h. At the end of 24 h, the starter culture reached a population of ~ 7 log CFU/ml. 5% of this culture was inoculated into 250 ml of production medium - GS and E. All flasks were incubated at 37° C on a rotary shaker (Innova 43) at 150 rpm for 96 h. Samples were taken at 0, 24, 48, 72 and 96 h for analysis of cell growth and nutrient consumption.

**2.4 Isolation of γ-PGA**

After 96 h, the cell suspension was centrifuged at 17000g for 30 minutes (Hermele 2 300K). Four volumes of cold 90% (v/v) ethanol was added to the cell free supernatant and incubated at 4°C for 72 h. Wet γ-PGA powder was obtained as sediment. The sediment was separated from the supernatant by centrifugation at 17000g for 30 mins. The obtained polymer was prepared for lyophilisation by dissolving it in 10 ml of deionised water in round bottom flasks. The flasks were then rotated gently on a mixture of 90% (v/v) ethanol at -20°C and dry ice to freeze the biopolymer in the form of a thin film. The frozen biopolymer was then lyophilized to obtain dry γ-PGA powder (Edward Modulo). The dried powder was weighed to calculate yield in g/l and stored in a desiccator for further analysis.

**2.5 Determination of growth**

To monitor cell growth, samples were taken aseptically at 0, 24, 48, 72 and 96h to monitor cell growth. Miles and Misra technique was used to calculate Colony Forming Units /ml (CFU/ml) in triplicates. Serial dilutions from 10-1 to 10-10 were performed by dispensing 0.5 ml of sample in 4.5 ml of sterile 1/4th strength ringer solution. 20 μl of each dilution was dispensed onto TSA plates under aseptic conditions. The plates were then incubated at 37°C for 24 h after which plates with less than 20 colonies were selected and used for viable count determination. CFU/ml was calculated with the formula:

$$CFU\!\!\Big/\!\!_{ml} = n \times \frac{1}{(Sample\ volume)} \times \frac{1}{D.F.}$$

: where n is the no. of colonies and D.F. is dilution factor.

**2.6 Chemical analysis**

Identification of γ-PGA

Isolated biopolymer was analyzed using Fourier Transformed Infra Red spectroscopy (FTIR) with an Impact 404 Nicolet spectrometer (UK) with KBr pellet in conjunction with OMNIC software. The FTIR spectra of the produced γ-PGA were compared with the spectra of a commercially available γ-PGA sample.

**Elemental analysis**

To evaluate if isolated polymer was in the form of free acid or salt, elemental analysis was performed using Inductively Coupled Plasma Atomic Emission Spectroscopy (ICP-AES) with SPECTRO CIROScco. Prior to the analysis, 6 ml HNO3 (70%) and 1 ml H2O2 (30% w/v) was added to 0.25 g of dried γ-PGA and this solution was digested with ETHOS 900 Microwave Labstation - Milestone Microwave Laboratory System.

**Molecular Weight**

Aqueous based gel permeation chromatography (GPC) was used to determine molecular weight (Mw), molecular number (Mn) and polydispersity (Smithers Rapra) using a MZ Hema guard plus 2 x Hema Linear column. 0.2 M NaNO3, 0.01 M NaH2PO4 at pH 7 was used as the eluent with a flow rate of 1.0 ml/min at 30°C and an RI detector. The data was collected and analyzed using Polymer Laboratories "Cirrus" software. GPC system used for this work was calibrated with sodium polyacrylate calibrants obtained from Polymer Laboratories.

**Nutrient consumption analysis**

Nutrient consumption analysis was performed using High Performance Liquid Spectroscopy (HPLC) with an HP series 1100 HPLC machine at the University of Reading, U.K. A Prevail Organic Acid 5u column with a UV detector was used for analyzing L-glutamic acid and citric acid whereas a Phenomenex carbohydrate column (Rezex RCM - monosaccharide Ca++ - 8%) was used for sucrose and glycerol determination. Filtered deionised water was used as the eluent for sucrose analysis. For organic acid analysis, 25 mM KH2PO4 (pH 2.5 adjusted with phosphoric acid) was used as the eluent. Before loading, the samples were filtered using 0.45 μm filters and were diluted 10 fold.

**Crystallinity**

Crystallinity was assessed using powder X-Ray Diffraction (XRD) analysis. Data was collected at room temperature with a Phillips PW1700, 40kV/40mA, CuKα instrument.

**Statistical analyses**

All results were analyzed using Microsoft Excel 2003. Two way Annova was used to compare data in Microsoft Excel 2003.

**3. Results and discussion**

**3.1 Growth**

Figures land 2 show the growth of all bacterial strains in both GS and E medium. At 0 h, all the strains had a cell count of ~5-6 CFU/ml. In GS medium, *B. subtilis* 23858 reached a maximum cell count of log 9.58 at 72 h. In medium E, *B. licheniformis* 1525 reached a maximum cell count of log 8.57 at 72 h. The maximum cell counts for the *B. subtilis* strains (~ log 7.5-8.1 CFU/ml) did not reach as high as that of the *B. licheniformis* strains (~ log 8.5 CFU/ml) in medium E. *B. subtilis* 23858 & 23859 and *B. licheniformis* 9945a, 1525 & 6816 had a higher cell count at 96 h in GS medium than in Medium E. *B. subtilis* 23859 did not grow well in medium E with a cell count of ~log 4.9 CFU/ml at the end of 96 h.

**3.2 Nutrient consumption**

Tables 3 and 4 below show utilization of nutrients by bacterial strains in both GS and E media after 96 h. In GS medium, except *B. subtilis* 23859 (75.71%), all strains utilized more than 85% of provided sucrose at the end of 96 h. In contrast, the C source in Medium E, i.e. glycerol, was not consumed as much (49.56-70.67%). In GS medium, *B.*

*subtilis* strains consumed more L-glutamic acid than *B. lic*heniformis strains. *B. subtilis* natto consumed the most L-glutamic acid at the end of 96 h ($\sim$95%). Incidentally, *B. subtilis* natto also produced the highest yield of $\gamma$-PGA in GS medium ($\sim$17.7g/l).

In contrast, in medium E, *B. licheniformis* strains consumed more L-glutamic acid than *B. subtilis* strains. This could possibly be reflected in the fact that the *B. licheniformis* strains produced a slightly higher yield of $\gamma$-PGA in medium E than most of the *B. subtilis* strains at the end of 96 h.

Since all the experiments were done in shake flasks, the pH and aeration of the culture could not be controlled which could have some impact on nutrient consumption.

**Table 3:** Nutrient utilization in GS medium after 96 h

| Strain Name | *B. subtilis 23856* | *B. subtilis 23857* | *B. subtilis 23858* | *B. subtilis 23859* | *B. subtilis* natto | *B. licheniformis 1525* | *B. licheniformis 6816* | *B. licheniformis 9945a* |
|---|---|---|---|---|---|---|---|---|
| % decrease in Sucrose | 90.66 | 88.19 | 90.77 | 75.71 | 89.97 | 94.77 | 97.96 | 86.98 |
| % decrease in L-glutamic acid | 64.72 | 58.48 | 82.26 | 67.25 | 95.01 | 39.22 | 33.84 | 17.46 |

Table 4: Nutrient utilization in Medium E after 96 h.

| Strain Name | *B. subtilis 23856* | *B. subtilis 23857* | *B. subtilis 23858* | *B. subtilis 23859* | *B. subtilis natto* | *B. licheniformis 1525* | *B. licheniformis 6816* | *B. licheniformis 9945a* |
|---|---|---|---|---|---|---|---|---|
| % decrease in Glycerol | 49.89 | 54.18 | 50.25 | 62.97 | 49.56 | 65.64 | 67.58 | 70.67 |
| % decrease in L-glutamic acid | 24.44 | 29.72 | 28.50 | 26.3 | 23.30 | 34.22 | 44.46 | 36.03 |

### 3.3 Identification

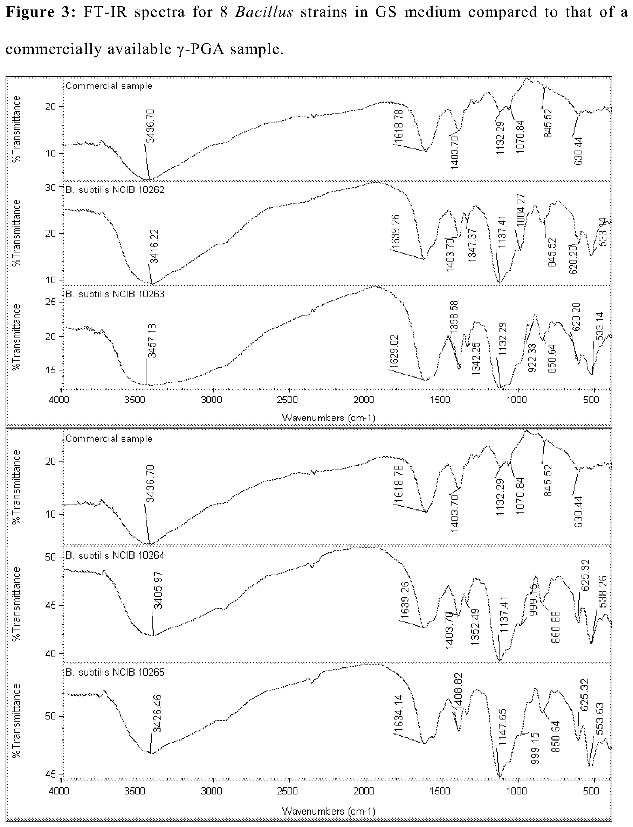

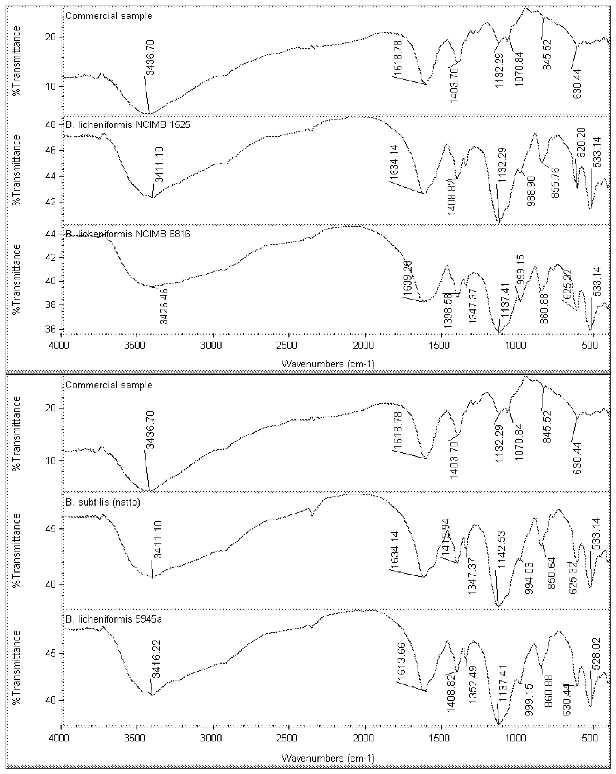

After isolation of γ-PGA, it was seen that cells grown in GS medium produced a white powder. In medium E, cells produced a brownish product, possibly due to the presence of ferric ions. The γ-PGA produced by all strains was identified by IR. All the strains showed similar results and the FT-IR spectra for γ-PGA obtained by the strains under investigation compared well to that of a commercially available γ-PGA sample (see Figure 3).

### 3.4 Yield

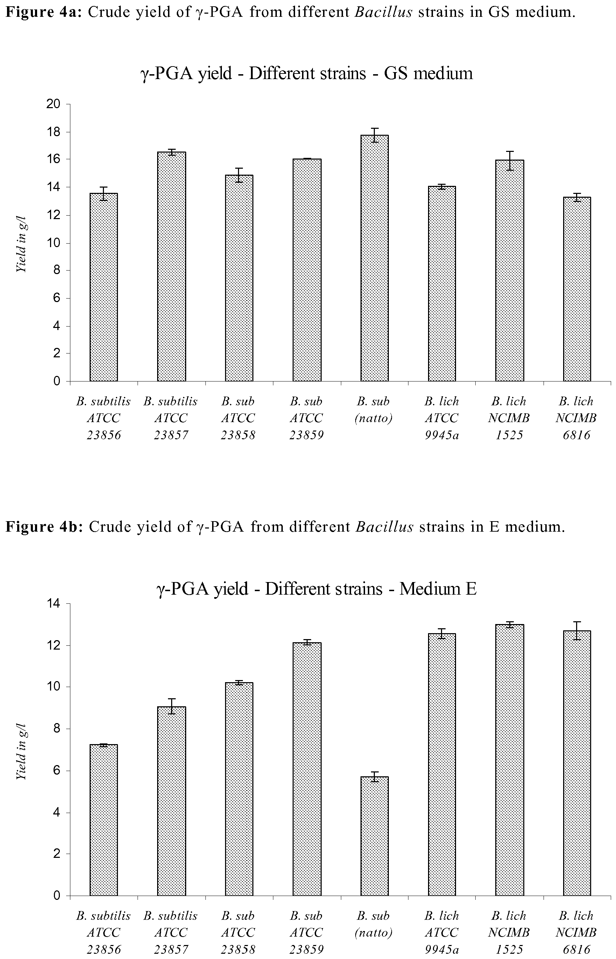

All strains produced more γ-PGA in GS medium than that in medium E, except in the case of *B. licheniformis* 9945a as shown in Figure 4. For *B. licheniformis* 9945a, the yields in both media are comparable. *B. subtilis* natto produced the maximum yield in GS medium (~17.7 g/l). This strain utilized more L-glutamic acid than any other strain in our study (~95%) and this could probably be reflected in the yield. However, the yields of the other strains do not necessarily correlate to the amount of L-glutamic acid consumed. Hence, the yield of γ-PGA could not only be dependent on the consumption of exogenous L-glutamic acid, but also on the ability of the bacteria to produce endogenous L-glutamic acid for production of γ-PGA. Medium E is known to be most suitable for production of γ-PGA with *B. licheniformis* 9945a, which is probably the most studied strain for the production of γ-PGA. All B. licheniformis strains produced more γ-PGA in medium E when compared to the *B. subtilis* strains, except *B. subtilis* 23859. *B. subtilis* 23859 consumed more glycerol in medium E than the other *B. subtilis* strains and this could be the reason why its yield of γ-PGA was slightly better. Since nutrient consumption could be affected by pH of the medium, the yield could also be affected.

### 3.5 Crystallinity

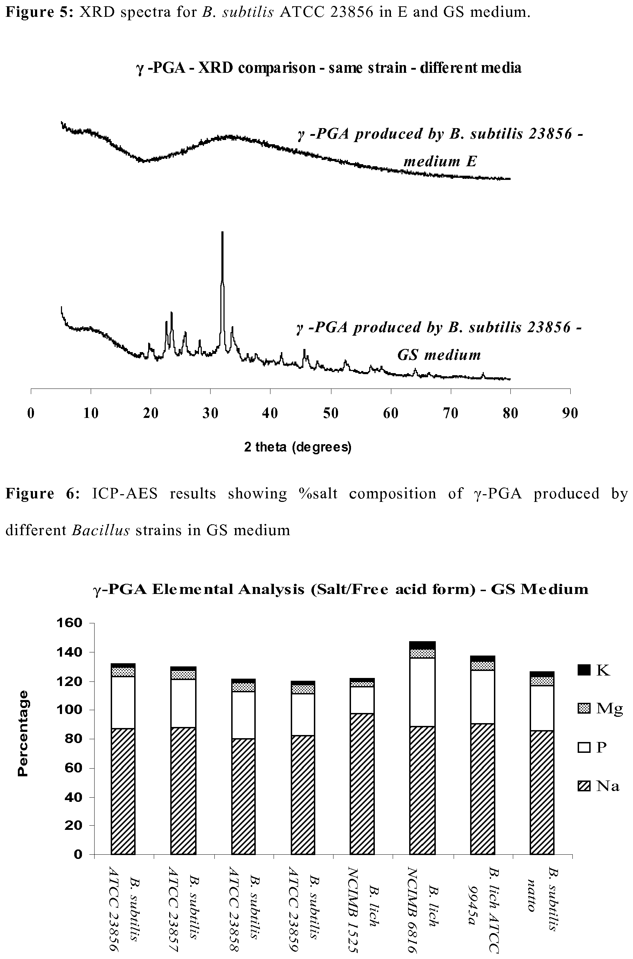

Amorphous γ-PGA is easily soluble in water, whereas a crystalline form of γ-PGA is relatively insoluble in water. XRD analysis showed that all strains produced amorphous γ-PGA with medium E. In contrast, a crystalline powder was obtained when the cells were grown in GS medium, which was evident because of the presence of distinct peaks on the spectra. The XRD spectra for one such strain - *B. subtilis* ATCC 23856 in GS and E medium has been shown in Figure 5.

### 3.6 Elemental analysis

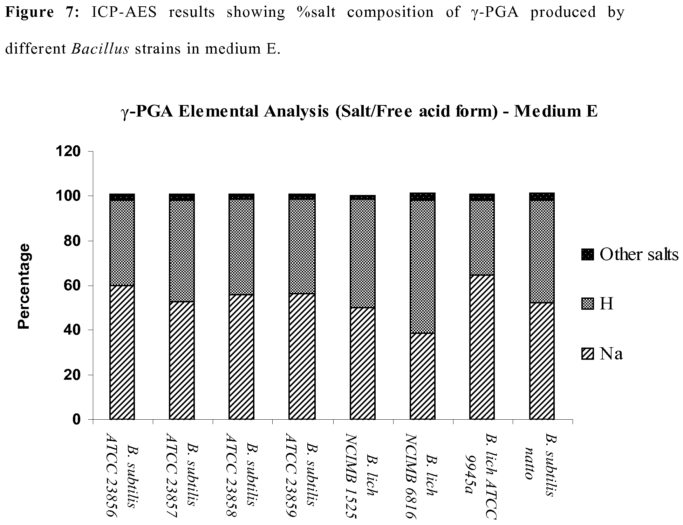

Elemental analysis was performed to identify whether the salt or the acid form of γ-PGA was produced. ICP-AES analysis breaks down the crude polymer and measures the concentration of individual elements that make up the polymer. Hence impurities, if present, in the sample would also be detected. ICP-AES results showed that most of the γ-PGA obtained with cells grown in GS medium was in fact the sodium salt of γ-PGA (Na-γ-PGA) with some γ-PGA also in its P, Mg and K salt form, see Figure 6. None of the strains produced the acid form of γ-PGA (H+-γ-PGA) in GS medium.
In contrast, strains grown in medium E produced considerable amount of H+-γ-PGA (37-57%) along with Na-γ-PGA, see Figure 7. The pH of medium E was adjusted with the help of 3M NaOH.
The free acid form of γ-PGA is insoluble in water, whereas the salt forms of γ-PGA are fully soluble in water.

### 3.7 Molecular weight

Molecular weight was determined with the help of aqueous based GPC. When grown in GS medium *B. licheniformis* NCIMB 1525 produced γ-PGA with a molecular weight of 871000 Da (polydispersity - 1.35) while *B. licheniformis* NCIMB 6816 produced γ-PGA with a molecular weight of 850000 Da (polydispersity - 1.45) and *B. subtilis* natto produced γ-PGA with a molecular weight of 257500 Da (polydispersity - 4.75). The other strains produced a lower molecular weight product (~3000 Da) With medium E, *B. licheniformis* NCIMB 6816 produced γ-PGA weighing 856500 Da (polydispersity - 1.2) and *B. licheniformis* 9945a produced γ-PGA weighing 760000 Da (polydispersity - 1.2) while the other strains produced a lower molecular weight product (~ <3000 Da).
A summary of the properties of γ-PGA obtained with the 8 Bacillus strains in both GS and E medium can be seen in Tables 5 and 6 below.

**Table 5:** Properties of γ-PGA produced from 8 *Bacillus* strains in GS medium

| Organism | Yield (g/l) | Crystallinity | % Na Salt | Molecular weight (Da) | Polydispersity |
|---|---|---|---|---|---|
| *B. subtilis 23856* | 13.55 ± 0.78 | Crystalline | 86.90 | ~ 3000 | n/a |
| *B. subtilis 23857* | 16.52 ± 0.35 | Crystalline | 87.55 | ~ 3000 | n/a |
| *B. subtilis 23858* | 14.88 ± 0.84 | Crystalline | 80.26 | ~ 3000 | n/a |

(continued)

| Organism | Yield (g/l) | Crystallinity | % Na Salt | Molecular weight (Da) | Polydispersity |
|---|---|---|---|---|---|
| *B. subtilis 23859* | 16.03 ± 0.06 | Crystalline | 82.40 | ~ 3000 | n/a |
| *B. licheniformis 1525* | 15.93 ± 1.16 | Crystalline | 97.40 | 871000 | 1.35 |
| *B. licheniformis 6816* | 13.3 ± 0.52 | Crystalline | 88.37 | 850000 | 1.45 |
| *B. subtilis* natto | 17.77 ± 0.9 | Crystalline | 85.38 | 257500 | 4.75 |
| *B. licheniformis* 9945a | 14.05 ± 0.31 | Crystalline | 90.30 | ~3000 | n/a |

**Table 6:** Properties of γ-PGA produced from 8 *Bacillus* strains in medium E.

| Organism | Yield (g/l) | Crystallinity | % Na Salt | %Acid form | Molecular weight (Da) | Polydispersity |
|---|---|---|---|---|---|---|
| *B. subtilis 23856* | 7.23 ± 0.07 | Amorphous | 59.90 | 37.76 | <3000 Da | n/a |
| *B. subtilis 23857* | 9.08 ± 0.34 | Amorphous | 52.68 | 44.98 | <3000 Da | n/a |
| *B. subtilis 23858* | 10.2 ± 0.09 | Amorphous | 56 | 41.91 | <3000 Da | n/a |
| *B. subtilis 23859* | 12.13 ± 0.12 | Amorphous | 56.29 | 41.73 | <3000 Da | n/a |
| *B. licheniformis 1525* | 12.98 ± 0.15 | Amorphous | 50.32 | 48.09 | <3000 Da | n/a |
| *B. licheniformis 6816* | 12.7 ± 0.45 | Amorphous | 38.84 | 57.97 | 856500 | 1.2 |
| *B. subtilis* natto | 5.7 ± 0.25 | Amorphous | 52.31394 | 44.5561 1 | <3000 Da | n/a |
| *B. licheniformis* 9945a | 12.58 ± 0.24 | Amorphous | 64.46865 | 32.8857 1 | 760000 Da | 1.2 |

## 4. Conclusion

This study produced γ-PGA using 8 strains of *Bacillus* and the γ-PGA produced had different properties (high/low molecular weight, amorphous/crystalline, salt and acid form).

If the cells are grown in GS medium, they produce a crystalline salt form of γ-PGA. In contrast, when they are grown in Medium E, an amorphous acid form of γ-PGA is produced. Crystallinity and form of γ-PGA seem to be *Bacillus* strain independent and these properties could be manipulated with the medium of production.

Molecular weight however, depends on the strain and medium of production. It can be seen from the results that *B. licheniformis* 6816 produced a very high molecular weight γ-PGA in both Medium E and GS, but *B. subtilis natto* produced a high molecular weight product only in GS medium. *B. licheniformis* 9945a produced a high molecular weight polymer in Medium E, but not in GS medium.

**PART 2. SUMMARY OF TESTS AFTER INVESTIGATING PRODUCTION OF γ-PGA USING 8 STRAINS OF BACILLUS**

**1. Introduction**

After producing γ-PGA using 8 different strains of *Bacillus* and characterizing its properties using various analytical techniques, *Bacillus subtilis* natto was chosen to produce γ-PGA for tests with probiotic bacteria.

**2. Materials and methods**

To produce γ-PGA for the tests, batch cultures of *B. subtilis* natto were carried out in a 5 litre fermenter. The bacteria were first inoculated in 250 ml of tryptone soy broth for 24 h at 37°C. 5% of this inoculum was added to fermenter. The fermentation temperature was maintained at 37°C, and the pH of the culture was allowed to drop naturally without the addition of acid. Once pH 6.5 was attained, the pH was maintained by automated addition of 3 M NaOH or 3 M HCl. The stirring speed and airflow were 250 rpm and 1.0 l/min respectively at the start of fermentation. Since γ-PGA is an extracellular polymer, the culture medium becomes highly viscous with increasing polymer production. The increased viscosity decreases the volumetric oxygen mass transfer, leading to oxygen limitation. The supply of oxygen was maintained above 40% by controlling the agitation speed and air flow rate. After 96 h, the cell suspension was centrifuged at 17000g for 30 minutes. Four volumes of cold 90% (v/v) ethanol was added to the cell free supernatant and incubated at 4°C for 72 h. Wet γ-PGA powder was obtained as sediment. The sediment was separated from the supernatant by centrifugation at 17000g for 30 mins. The wet crude polymer was then dissolved in water and dialyzed to eliminate impurities lower than 10,000 Da. The obtained pure polymer was prepared for lyophilisation in round bottom flasks. The flasks were rotated gently on a mixture of 90% (v/v) ethanol at -20°C and dry ice to freeze the biopolymer in the form of a thin film. The frozen biopolymer was then lyophilized to obtain dry γ-PGA powder. The pure dried powder was weighed to calculate yield in g/l and stored in a desiccator for tests with probiotics.

**3. Tests with probiotics**

**3.1 γ-PGA as a cryoprotectant**

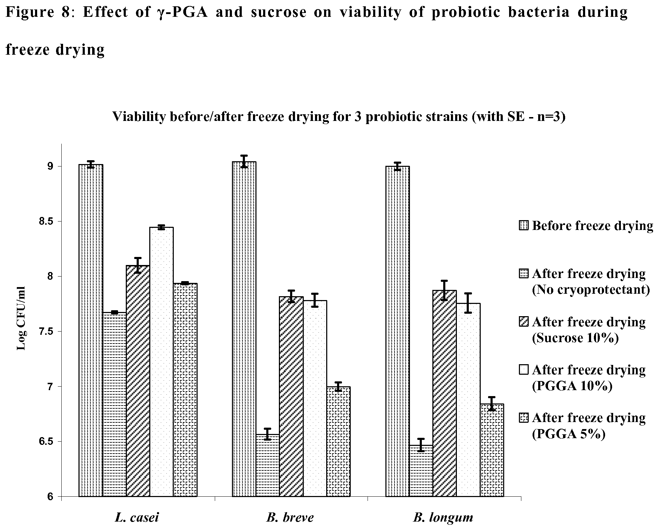

Three probiotic bacteria, *Bifidobacterium longum, Bifidobacterium breve* and *Lactobacillus casei* were used for the tests. The effect of 10% Na-γ-PGA was tested on viability of the bacteria before and after freeze drying. To coat cells with cryoprotectant before freeze drying, *Bifidobacteria* were inoculated in TPY broth (22 h for *B. breve* and 16 h for *B. longum*) and *Lactobacillus casei* was inoculated in MRS broth (for 48 h) at 37 °C anaerobically. After incubation, viable counts were taken on TPY agar (*Bifidobacteria*) and MRS agar (*Lactobacillus*) to determine number of viable cells before freeze drying. The cultures were then centrifuged and washed with PBS to obtain cell pellets. Cells were then mixed thoroughly in 10 ml solutions of 10% Na-γ-PGA, 5% Na-γ-PGA and 10% sucrose. For cells without a cryoprotectant, 10 ml of water was added. Cells were then frozen at -80 °C for 24 h and freeze dried to obtain a dry powder. After freeze drying, 10 ml of PBS was added to all the cells and viability was measured to determine number of viable cells after freeze drying. All tests were done in triplicates. The results, shown in Figure 8, showed that for all three bacteria, Na-γ-PGA was able to improve viability, compared to when no cryoprotectant was used. 10% Na-γ-PGA was a better cryoprotectant than 10% sucrose for *L. casei,* whereas its cryoprotective ability was comparable to 10% sucrose for both *Bifidobacteria* strains.

**3.2. Na-γ-PGA as a protectant in fruit juice and simulated gastric juice**

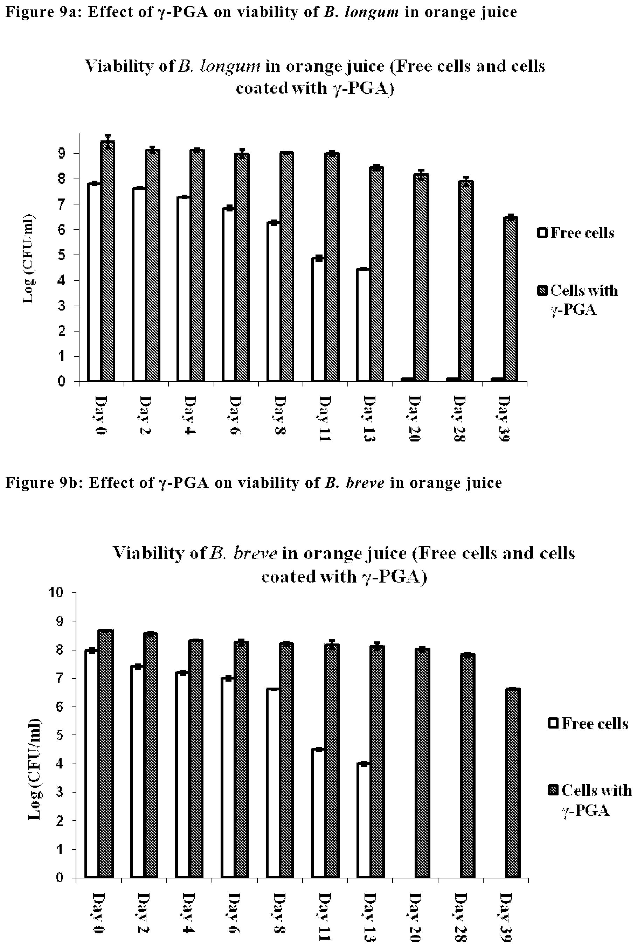

The effect of 2.5% Na-γ-PGA was tested on viability of the two *Bifidobacteria* strains when stored in orange juice for 39 days and simulated gastric juice for 4 h. To coat cells with Na-γ-PGA, bacteria were inoculated in TPY broth (22 h for *B. breve* and 16 h for *B. longum*) at 37 °C. The culture was then centrifuged and washed with PBS to obtain cell pellets. Cells were then mixed thoroughly in a 10% Na-γ-PGA solution (1 gm Na-γ-PGA in 9 ml of deionised water). This mixture was frozen at -80 °C and freeze dried to obtain a dry powder with cells coated with Na-γ-PGA. 1 ml PBS was added to the dry cells and this solution was transferred to 40 ml of orange juice. Final concentration of Na-γ-PGA in fruit juice was ∼ 2.5 %. For cells without γ-PGA coating, cells were inoculated in TPY broth under the aforementioned conditions. Cell pellets were obtained after centrifugation and washing with PBS. 1 ml of PBS was added to the cells. This solution was added to orange juice. Viability was measured at day 0, 2, 4, 6, 8, 11, 13, 20, 28 and 39 for cells with and without Na-γ-PGA coating on Bifidobacteria Selective Medium Agar (BSM agar). The results are shown in Figure 9.
Cells that were not coated with Na-γ-PGA failed to survive in orange juice for 20 days. In contrast, cells with a Na-γ-PGA coating survived well for 39 days with a viability of about log 6-7 CFU/ml, when compared to their initial count of log 8-9 CFU/ml. Hence, Na-γ-PGA can be used to noticeably increase the shelf life of the food probiotic product.

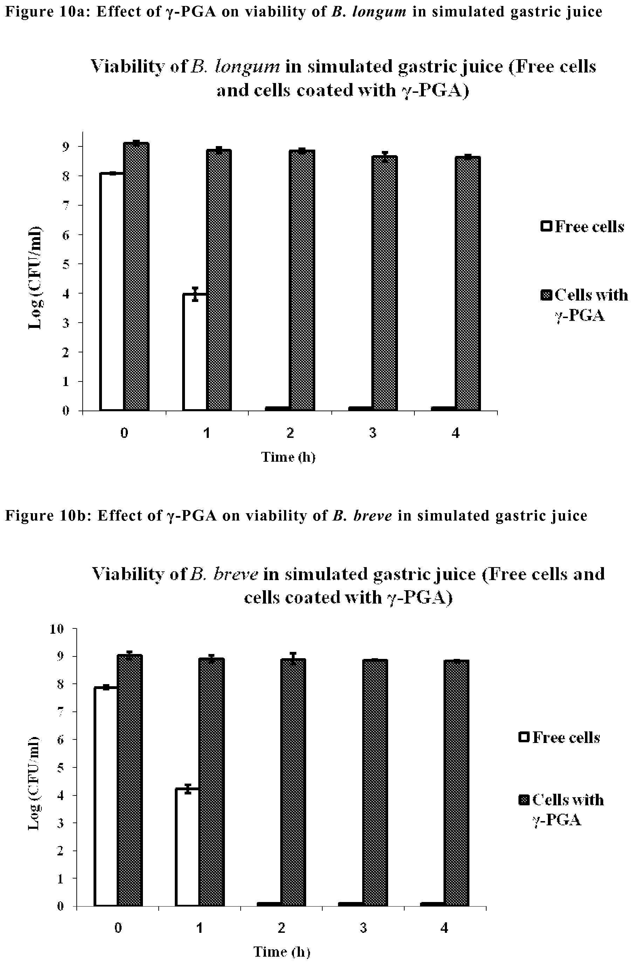

Similarly the effect of 2.5% Na-γ-PGA was tested on viability of the 2 *Bifidobacteria* strains when stored in simulated gastric juice for 4 h. Simulated gastric juice with pH 2.0 was prepared in the lab to mimic the environment of the stomach. The aforementioned protocol was followed to coat cells with Na-γ-PGA. Viability was measured at 0h, 1h, 2h, 3h and 4h for cells with and without Na-γ-PGA coating on BSM agar. The results are shown in Figures 10. For cells that were coated with Na-γ-PGA, there was no noticeable loss in viability. However, cells without Na-γ-PGA coating died within 2 hours.

**Claims**

1.  A probiotic microorganism at least partially coated directly with a poly glutamic acid, wherein the poly glutamic acid is poly-γ-glutamic acid (γ-PGA).

2.  The probiotic microorganism of claim 1 wherein the γ-PGA has a molecular weight of from 10,000 to 1,000,000; wherein optionally,
    the γ-PGA has a molecular weight of from 20,000 to 800,000; wherein more optionally,
    the γ-PGA has a molecular weight of from 100,000 to 300,000.

3.  The probiotic microorganism of any of the preceding claims wherein the γ-PGA is produced in *Bacillus subtilis* or *Bacillus licheniformis;* wherein optionally,
    the γ-PGA is produced in *Bacillus subtilis* natto.

4.  The probiotic microorganism of any of the preceding claims wherein the probiotic microorganism is a bacteria.

5.  An ingestible product comprising a probiotic microorganism of any of claims 1 to 4.

6.  The ingestible product of claim 5 wherein all or part, of some or all, of the probiotic microorganisms is coated with a poly-γ-glutamic acid.

7.  The ingestible product of claim 5 or claim 6 wherein the ingestible product is a food or beverage product.

8.  The ingestible product of any of claims 5 to 7 wherein the ingestible product is milk based.

9.  The ingestible product of any of claims 5 to 7 wherein the ingestible product is non-milk based.

10. A method of at least partially coating directly a probiotic microorganism with a poly glutamic acid comprising mixing the microorganism with a solution of a poly glutamic acid, wherein the poly glutamic acid is poly-γ-glutamic acid (γ-PGA).

11. The method of claim 10 wherein the poly glutamic acid is provided in a concentration of from 2 to 15% (w/v).

12. The method of claim 10 or claim 11 wherein the microorganism is provided in cell pellet form.

13. The method of any of claims 10 to 12 wherein the γ-PGA has a molecular weight of from 10,000 to 1,000,000; wherein optionally,
    the γ-PGA has a molecular weight of from 20,000 to 800,000; wherein more optionally,
    the γ-PGA has a molecular weight of from 100,000 to 300,000.

14. The method of any of claims 10 to 13 wherein the γ-PGA is produced in *Bacillus subtilis* or *Bacillus licheniformis*; wherein optionally,
    the γ-PGA is produced in *Bacillus subtilis* natto.

15. The method of any of claims 10 to 14 wherein the probiotic microorganism is a bacteria.

**Patentansprüche**

1.  Probiotischer Mikroorganismus, der wenigstens teilweise direkt mit einer Polyglutaminsäure beschichtet ist, wobei die Polyglutaminsäure Poly-γ-glutaminsäure (γ-PGA) ist.

**2.** Probiotischer Mikroorganismus gemäß Anspruch 1, wobei die γ-PGA ein Molekulargewicht von 10.000 bis 1.000.000 aufweist; wobei gegebenenfalls
die γ-PGA ein Molekulargewicht von 20.000 bis 800.000 aufweist; wobei weiter gegebenenfalls
die γ-PGA ein Molekulargewicht von 100.000 bis 300.000 aufweist.

**3.** Probiotischer Mikroorganismus gemäß einem der vorstehenden Ansprüche, wobei die γ-PGA in *Bacillus subtilis* oder *Bacillus licheniformis* hergestellt ist; wobei gegebenenfalls
die γ-PGA in *Bacillus subtilis* natto hergestellt ist.

**4.** Probiotischer Mikroorganismus gemäß einem der vorstehenden Ansprüche, wobei der probiotische Mikroorganismus ein Bakterium ist.

**5.** Verzehrbares Produkt, umfassend einen probiotischen Mikroorganismus gemäß einem der Ansprüche 1 bis 4.

**6.** Verzehrbares Produkt gemäß Anspruch 5, wobei alles oder ein Teil von manchen oder allen der probiotischen Mikroorganismen mit einer Poly-γ-glutaminsäure beschichtet ist.

**7.** Verzehrbares Produkt gemäß Anspruch 5 oder Anspruch 6, wobei das verzehrbare Produkt ein Lebensmittel- oder Getränkeprodukt ist.

**8.** Verzehrbares Produkt gemäß einem der Ansprüche 5 bis 7, wobei das verzehrbare Produkt auf Milchbasis ist.

**9.** Verzehrbares Produkt gemäß einem der Ansprüche 5 bis 7, wobei das verzehrbare Produkt auf Nichtmilchbasis ist.

**10.** Verfahren zum wenigstens teilweisen direkten Beschichten eines probiotischen Mikroorganismus mit einer Polyglutaminsäure, umfassend Mischen des Mikroorganismus mit einer Lösung einer Polyglutaminsäure, wobei die Polyglutaminsäure Poly-γ-glutaminsäure (γ-PGA) ist.

**11.** Verfahren gemäß Anspruch 10, wobei die Polyglutaminsäure mit einer Konzentration von 2 bis 15 % (w/v) bereitgestellt wird.

**12.** Verfahren gemäß Anspruch 10 oder 11, wobei der Mikroorganismus in Zellpelletform bereitgestellt wird.

**13.** Verfahren gemäß einem der Ansprüche 10 bis 12, wobei die γ-PGA ein Molekulargewicht von 10.000 bis 1.000.000 aufweist; wobei gegebenenfalls die γ-PGA ein Molekulargewicht von 20.000 bis 800.000 aufweist; wobei weiter gegebenenfalls
die γ-PGA ein Molekulargewicht von 100.000 bis 300.000 aufweist.

**14.** Verfahren gemäß einem der Ansprüche 10 bis 13, wobei die γ-PGA in *Bacillus subtilis* oder *Bacillus licheniformis* hergestellt ist; wobei gegebenenfalls
die γ-PGA in *Bacillus subtilis* natto hergestellt ist.

**15.** Verfahren gemäß einem der Ansprüche 10 bis 14, wobei der probiotische Mikroorganismus ein Bakterium ist.

**Revendications**

**1.** Micro-organisme probiotique au moins en partie revêtu directement d'un acide polyglutamique, dans lequel l'acide polyglutamique est l'acide poly-γ-glutamique (γ-PGA).

**2.** Micro-organisme probiotique selon la revendication 1, dans lequel l'γ-PGA a une masse moléculaire de 10 000 à 1 000 000 ; dans lequel, optionnellement,
l'γ-PGA a une masse moléculaire de 20 000 à 800 000 ; dans lequel en outre, optionnellement,
l'γ-PGA a une masse moléculaire de 100 000 à 300 000.

**3.** Micro-organisme probiotique selon l'une quelconque des revendications précédentes, dans lequel l'γ-PGA est produit dans le *Bacillus subtilis* ou le *Bacillus licheniformis ;* dans lequel, optionnellement, l'γ-PGA est produit dans le *Bacillus subtilis natto.*

4. Micro-organisme probiotique selon l'une quelconque des revendications précédentes, le micro-organisme probiotique étant une bactérie.

5. Produit ingérable comprenant un micro-organisme probiotique selon l'une quelconque des revendications 1 à 4.

6. Produit ingérable selon la revendication 5, dans lequel la totalité ou une partie, de certains ou de tous les micro-organismes probiotiques, est revêtue d'un acide poly-γ-glutamique.

7. Produit ingérable selon la revendication 5 ou la revendication 6, le produit ingérable étant un produit sous forme d'aliment ou de boisson.

8. Produit ingérable selon l'une quelconque des revendications 5 à 7, le produit ingérable étant à base de lait.

9. Produit ingérable selon l'une quelconque des revendications 5 à 7, le produit ingérable n'étant pas à base de lait.

10. Procédé de revêtement au moins en partie et directement d'un micro-organisme probiotique avec un acide polyglutamique, comprenant le mélange du micro-organisme avec une solution d'un acide polyglutamique, l'acide polyglutamique étant l'acide poly-γ-glutamique (γ-PGA).

11. Procédé selon la revendication 10, dans lequel l'acide polyglutamique est présent à une concentration de 2 à 15 % (p/v).

12. Procédé selon la revendication 10 ou la revendication 11, dans lequel le micro-organisme est présent sous forme de granules de cellules.

13. Procédé selon l'une quelconque des revendications 10 à 12, dans lequel l'γ-PGA a une masse moléculaire de 10 000 à 1 000 000 ; dans lequel, optionnellement, l'γ-PGA a une masse moléculaire de 20 000 à 800 000 ; dans lequel en outre, optionnellement, l'γ-PGA a une masse moléculaire de 100 000 à 300 000.

14. Procédé selon l'une quelconque des revendications 10 à 13, dans lequel l'γ-PGA est produit dans le *Bacillus subtilis* ou le *Bacillus licheniformis ;* dans lequel, optionnellement, l'γ-PGA est produit dans le *Bacillus subtilis natto.*

15. Procédé selon l'une quelconque des revendications 10 à 14, dans lequel le micro-organisme probiotique est une bactérie.

**Figure 1**: Growth of *Bacillus* strains in GS medium

Growth curve - *Bacillus* strains - GS medium

**Figure 2**: Growth of *Bacillus* strains in Medium E

Growth curve - *Bacillus* strains - Medium E

**Figure 3:** FT-IR spectra for 8 *Bacillus* strains in GS medium compared to that of a commercially available γ-PGA sample.

**Figure 4a:** Crude yield of γ-PGA from different *Bacillus* strains in GS medium.

γ-PGA yield - Different strains - GS medium

**Figure 4b:** Crude yield of γ-PGA from different *Bacillus* strains in E medium.

γ-PGA yield - Different strains - Medium E

**Figure 5:** XRD spectra for *B. subtilis* ATCC 23856 in E and GS medium.

γ -PGA - XRD comparison - same strain - different media

γ -PGA produced by B. subtilis 23856 - medium E

γ -PGA produced by B. subtilis 23856 - GS medium

2 theta (degrees)

**Figure 6:** ICP-AES results showing %salt composition of γ-PGA produced by different *Bacillus* strains in GS medium

γ-PGA Elemental Analysis (Salt/Free acid form) - GS Medium

**Figure 7:** ICP-AES results showing %salt composition of γ-PGA produced by different *Bacillus* strains in medium E.

γ-PGA Elemental Analysis (Salt/Free acid form) - Medium E

Figure 8: Effect of γ-PGA and sucrose on viability of probiotic bacteria during freeze drying

Viability before/after freeze drying for 3 probiotic strains (with SE - n=3)

**Figure 9a: Effect of γ-PGA on viability of *B. longum* in orange juice**

Viability of *B. longum* in orange juice (Free cells and cells coated with γ-PGA)

**Figure 9b: Effect of γ-PGA on viability of *B. breve* in orange juice**

Viability of *B. breve* in orange juice (Free cells and cells coated with γ-PGA)

**Figure 10a: Effect of γ-PGA on viability of *B. longum* in simulated gastric juice**

Viability of *B. longum* in simulated gastric juice (Free cells and cells coated with γ-PGA)

**Figure 10b: Effect of γ-PGA on viability of *B. breve* in simulated gastric juice**

Viability of *B. breve* in simulated gastric juice (Free cells and cells coated with γ-PGA)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101000364

**Non-patent literature cited in the description**

- **SHANTANU S. BALKUNDI et al.** *LANGMUIR,* 15 December 2009, vol. 25 (24